(19) Europäisches Patentamt
European Patent Office
Office européen des brevets

(11) **EP 1 428 522 B1**

(12) **FASCICULE DE BREVET EUROPEEN**

(45) Date de publication et mention
de la délivrance du brevet:
**27.09.2006 Bulletin 2006/39**

(51) Int Cl.:
*A61K 8/19* (2006.01)    *A61K 8/60* (2006.01)
*A61Q 19/00* (2006.01)    *A61Q 19/08* (2006.01)

(21) Numéro de dépôt: **03292552.1**

(22) Date de dépôt: **14.10.2003**

(54) **Composition cosmétique, comprenant l'adénosine et des sels de magnésium et potassium**

Kosmetische Zusammensetzung enthaltend Adenosin sowie Magnesium- und Kaliumsalze

Cosmetic composition comprising adenosine and magnesium and potassium salts

(84) Etats contractants désignés:
**AT BE BG CH CY CZ DE DK EE ES FI FR GB GR HU IE IT LI LU MC NL PT RO SE SI SK TR**

(30) Priorité: **26.11.2002 FR 0214829**

(43) Date de publication de la demande:
**16.06.2004 Bulletin 2004/25**

(73) Titulaire: **L'ORÉAL**
**75008 Paris (FR)**

(72) Inventeurs:
• **Galey, Jean-Baptiste**
**93600 Aulnay-sous-Bois (FR)**
• **Hirt, Jean-Pascal**
**92210 Saint-Cloud (FR)**

(74) Mandataire: **Renard, Emmanuelle**
**L'OREAL - D.I.P.I.**
**25-29 Quai Aulagnier**
**92600 Asnières (FR)**

(56) Documents cités:
WO-A-01/43704          FR-A- 1 440 795
US-B1- 6 423 327

• **PATENT ABSTRACTS OF JAPAN vol. 1998, no. 05, 30 avril 1998 (1998-04-30) & JP 10 007541 A (NOEVIR CO LTD), 13 janvier 1998 (1998-01-13)**
• **DATABASE WPI Section Ch, Week 200279 Derwent Publications Ltd., London, GB; Class A96, AN 2002-726494 XP002251273 & JP 2002 212052 A (ITO H) 31 juillet 2002 (2002-07-31)**

EP 1 428 522 B1

**Description**

La présente invention concerne une composition renfermant, dans un milieu physiologiquement acceptable adapté à une application topique sur la peau, l'adénosine, au moins un sel de magnésium et au moins un sel de potassium. Elle concerne également un procédé cosmétique pour réduire les rides, notamment d'expression, et/ou détendre les traits et/ou décontracter la peau, comprenant l'application topique sur la peau de cette composition.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané ou en prévenant sa dégradation.

Bien que ces traitements permettent d'agir sur les rides et ridules dues au vieillissement chronologique ou intrinsèque, ainsi que de celles dues au photo-vieillissement, ils n'ont pas d'effet sur les rides d'expression.

Les rides d'expression sont en effet la résultante de mécanismes différents de ceux générant les rides dues au vieillissement.

Précisément, elles sont produites sous l'effet de la contrainte exercée sur la peau par les muscles peauciers qui permettent les mimiques. Selon la forme du visage, la fréquence des mimiques et les tics éventuels, elles peuvent apparaître dès l'enfance. L'âge, de même que certains facteurs environnementaux tels que l'exposition au soleil, n'intervient pas dans leur genèse mais peut les creuser davantage et les rendre permanentes.

Les rides d'expression se caractérisent par la présence de sillons sur le pourtour des orifices que constituent le nez (sillons nasogéniens), la bouche (rides para-buccales et rides dites de l'amertume) et les yeux (rides de la patte d'oie), autour desquels se situent les muscles peauciers, ainsi qu'entre les sourcils (rides de la glabelle ou du lion) et sur le front.

Jusqu'à présent, les seuls moyens couramment utilisés pour agir sur les rides d'expression sont, d'une part, la toxine botulique qui est notamment injectée dans les rides de la glabelle (voir J.D. Carruters et al., J. Dermatol. Surg. Oncol., 1992, 18, pp. 17-21) et, d'autre part, des implants dégradables à base de collagène, d'acide hyaluronique ou d'acide polylactique.

En outre, comme alternative à ces techniques médicales nécessitant le recours à un praticien, la Demanderesse a proposé divers composés susceptibles d'offrir un effet myorelaxant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681 ), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits d'Iris pallida (FR-2 746 641).

Il reste toutefois le besoin de disposer de composés efficaces pour lisser ou estomper les rides d'expression.

Or, la Demanderesse a découvert avec étonnement que l'utilisation conjointe d'adénosine et de sels de magnésium et de potassium permettait de satisfaire ce besoin. Précisément, il a été démontré que l'association de l'adénosine avec ces sels permettait de relaxer ou décontracter significativement les cellules contractiles dermiques qui sont supposées être impliquées dans la genèse des rides d'expression. On pense en effet que le phénotype de certains fibroblastes situés le long des lignes de tension créées sous l'effet des contractions des muscles peauciers lors des mimiques serait progressivement modifié sous l'effet de ces contractions, conférant ainsi à ces fibroblastes des propriétés contractiles particulières. La décontraction de ces cellules permettrait ainsi de lutter contre les rides d'expression.

Il a été suggéré dans le document US-6,423,327 d'utiliser l'adénosine ou un analogue d'adénosine, dans une composition appliquée topiquement sur la peau -qui peut comprendre divers adjuvants dont du sorbate de potassium-, en vue d'améliorer l'état de la peau et en particulier pour lutter contre les rides, le relâchement cutané, la sécheresse de la peau et les taches pigmentaires. Il est indiqué que l'adénosine a pour effet d'augmenter la taille des fibroblastes, ainsi que la synthèse de protéines par les fibroblastes.

En revanche, il n'est pas suggéré d'ajouter un sel de magnésium à la composition à base d'adénosine divulguée dans ce document.

En outre, il n'est pas suggéré dans ce document que l'adénosine puisse avoir un quelconque effet sur les rides d'expression ni sur la relaxation des fibroblastes contractiles. Au contraire, le traitement des rides d'expression passe par une décontraction du visage, alors que le document US-6,423,327 vise à raffermir la peau pour lutter contre le relâchement cutané.

La demande WO 00/56145 divulgue une composition pharmaceutique anesthésiante destinée à protéger des organes, plus particulièrement le coeur mais aussi éventuellement la peau, lors d'interventions chirurgicales, qui comprend notamment de l'adénosine et/ou un ouvreur de canaux potassiques qui peut être un antagoniste calcique. Toutefois, le magnésium n'est pas cité comme exemple d'antagoniste calcique dans cette demande. En outre, il n'est pas suggéré d'utiliser cette composition dans le traitement des rides ou sur une peau ridée.

Enfin, le document FR-1 440 795 décrit une composition cosmétique comprenant l'adénosine, l'AMP, l'ADP ou l'ATP. Ces phosphates peuvent se trouver sous forme de sels, notamment de magnésium ou de potassium. En revanche, il n'est pas décrit de composition associant l'adénosine elle-même à des sels de potassium et de magnésium. En outre, il n'est pas suggéré d'utiliser cette composition dans le traitement des rides ou sur une peau ridée.

La présente invention a donc pour objet une composition renfermant, dans un milieu physiologiquement acceptable adapté à une application topique sur la peau, l'adénosine, au moins un sel de magnésium et au moins un sel de potassium.

Elle a également pour objet un procédé cosmétique pour réduire les rides, notamment d'expression, et/ou détendre les traits et/ou décontracter la peau, comprenant l'application topique sur la peau de cette composition.

L'adénosine utilisée dans la présente invention est notamment disponible dans le commerce sous forme de poudre auprès de la société PHARMA WALDHOF.

Comme sels de magnésium et de potassium, on peut citer les sels organiques et inorganiques de ces métaux.

Des exemples de sels organiques de magnésium et de potassium comprennent les sels formés à partir d'un contre-anion choisi parmi : un anion citrate, oxalate, acétate, gluconate, lactate, tartrate, maléate, benzoate, propionate, salicylate, ascorbate, formate, succinate, folinate, aspartate, phthalate, oléate, palmitate, stéarate, lauryl sulfate, lanolate, myristate, béhénate, caséinate, cyclamate, pantothénate, polyaminopolycarboxylate, thioglycolate, laurate, ricinoléate, pidolate, sorbate ou glycyrrhizinate.

Des exemples de sels inorganiques de magnésium et de potassium comprennent les sels formés à partir d'un contre-anion choisi parmi : un anion nitrate, sulfate, halogénure, carbonate, bicarbonate, hydroxyde, peroxyde, nitrure, sulfure, bisulfate, persulfate, glycérophosphate, hypophosphate ou borate.

Le sulfate de magnésium et le glycyrrhizinate dipotassique sont préférés pour une utilisation dans la présente invention.

Les quantités d'adénosine et de sels qui peuvent être introduites dans la composition selon l'invention peuvent varier dans une large mesure en fonction de l'effet recherché. A titre d'exemple, l'adénosine peut représenter de 0,001 à 5% et de préférence de 0,01 à 1% du poids total de la compsoition. Le sel de magnésium peut représenter de 0,001 à 1% et de préférence de 0,01 à 0,1% du poids total de la composition. Le sel de potassium peut représenter de 0,001 à 1% et de préférence de 0,01 à 0,1% du poids total de la composition.

La composition selon l'invention est plus particulièrement destinée à être appliquée sur les zones du visage ou du front marquées par des rides d'expression, et/ou sur les personnes présentant des rides d'expression.

Les rides concernées sont de préférence choisies parmi : les rides de la patte d'oie, les sillons nasogéniens, les rides inter-sourcillières et les rides du front.

La composition selon l'invention peut être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage pour la peau.

La composition selon l'invention peut se présenter sous toutes les formes galéniques normalement utilisées dans le domaine cosmétique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Lorsque la composition utilisée selon l'invention est une émulsion, la proportion de la phase grasse peut aller de 5 à 80% en poids, et de préférence de 5 à 50% en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30% en poids, et de préférence de 0,5 à 20% en poids par rapport au poids total de la composition.

Comme huiles utilisables dans l'invention, on peut citer les hydrocarbures d'origine minérale ou synthétique (huile de vaseline, isohexadécane), les huiles d'origine végétale (huile d'amande d'abricot, fraction liquide de beurre de karité, d'avocat, huile de soja), les huiles d'origine animale (lanoline), les huiles de synthèse (perhydrosqualène, tétraoctanoate de pentaérythrityle), les huiles siliconées (cyclopentasiloxane et cyclohexasiloxane) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille).

Comme émulsionnants et coémulsionnants utilisables dans l'invention, on peut citer par exemple les esters d'acide gras et de polyéthylène glycol tels que le stéarate dé PEG-100 et le stéarate de PEG-20 et les esters d'acide gras et de glycérine tels que le stéarate de glycéryle.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de l'association d'actifs selon l'invention.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme conservateurs, on peut citer les esters d'acide para-hydroxybenzoïque, l'octane 1,2-diol, l'iodo-3 propynyl-2 butyl carbamate, le phénoxyéthanol et le gluconate de chlorhexidine.

Comme charges, on peut citer par exemple, les particules de polyamide (Nylon® ) ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

Comme indiqué précédemment, la composition selon l'invention peut également renfermer des filtres UVA et/ou UVB, sous forme de composés organiques ou inorganiques, ces derniers étant éventuellement enrobés pour les rendre hydrophobes.

Les filtres organiques plus particulièrement préférés sont choisis parmi les composés suivants (cités selon la nomenclature CTFA) : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, ainsi que le 1,1-dicarboxy (2,2'-diméthyl-propyl)-4,4-diphénylbutadiène, et leurs mélanges.

Les filtres inorganiques sont de préférence constitués d'oxyde de zinc, de fer, de zirconium, de cérium et/ou de titane (amorphe ou cristallisé sous forme rutile et/ou anatase), de préférence de taille nanométrique (taille moyenne des particules primaires: généralement entre 5 nm et 100 nm, de préférence entre 10 nm et 50 nm), éventuellement enrobés d'alumine et/ou d'acide stéarique.

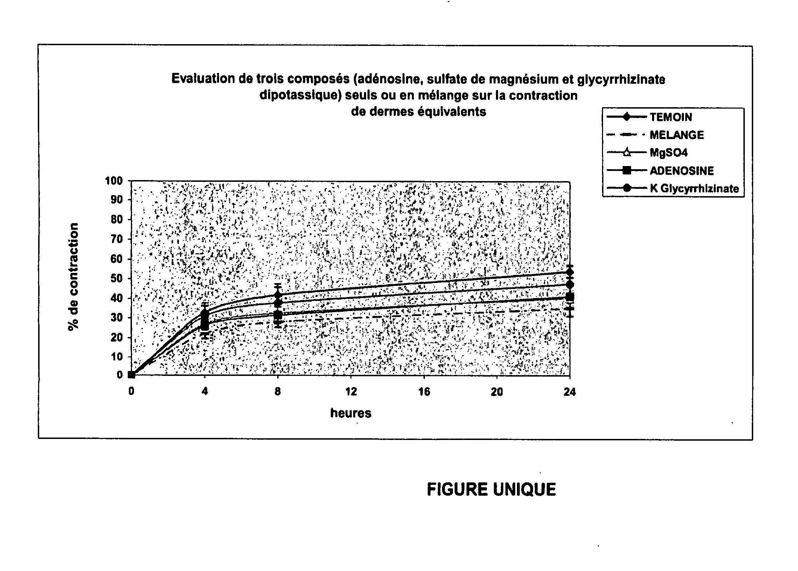

L'invention sera maintenant illustrée par les exemples non limitatifs suivants. Dans ces exemples, il est fait référence à la Figure annexée qui illustre la contraction au cours du temps d'un derme équivalent traité par les constituants de la composition selon l'invention.

**EXEMPLES**

**Exemple 1 : Mise en évidence *in vitro* de l'effet dermo-décontractant**

a) Principe du test

Le principe de ce test a consisté à étudier l'effet relaxant d'une association d'adénosine, de sel de potassium et de sel de magnésium sur un modèle de derme équivalent constitué d'une matrice de collagène ensemencée par des fibroblastes humains normaux.

Ces conditions sont destinées à mimer *in vitro* les phénomènes contractiles dermiques qui se produisent lors des mimiques du visage. Dans ces conditions, en effet, les cellules expriment spontanément des forces de traction qui induisent une rétraction du gel de collagène. Il en résulte une diminution de la surface totale du derme équivalent au cours du temps. La mesure de cette surface permet d'évaluer les effets de relaxation des substances préalablement mises en contact avec le derme équivalent.

b) Protocole

Cinq séries de trois dermes équivalents attachés contenant des fibroblastes humains normaux sont préparés :

une série témoin sans aucun traitement, et quatre séries respectivement traitées par l'adénosine (0,01%), le sulfate de magnésium (0,05%), le glycyrrhizinate dipotassique (quantité suffisante pour disposer de 0,05% de potassium dans le milieu de culture) et le mélange de ces trois composés dans les proportions indiquées. L'expérience est répétée trois fois.

Les équivalents de derme sont préparés comme décrits dans Asselineau et col., Exp. Cell. Res., 1985, 159, 536-539 ; Models in dermatology, 1987, vol 3 pp 1-7, dans les proportions suivantes :

| | |
|---|---|
| Milieu MEM (1,76X) avec ou sans composé(s) testé(s) | 45% |
| Sérum de Veau Foetal : | 9% |
| NaOH (0.1N) : | 5% |
| Acide acétique (1/1000) : | 4% |
| Collagène : | 26% |
| Fibroblastes : | 11 % |

Le collagène utilisé est du collagène de type 1 (solution commerciale), mais on peut également utiliser du collagène de type III ou IV. Il est extrait de queues de rat ou de peau de veau par hydrolyse acide et conservé en milieu acide à +4°C ; il polymérise naturellement par réchauffement à 37°C et par diminution du taux d'acidité. Le collagène est préalablement dialysé contre des bains successifs d'eau + acide acétique.

Le protocole est le suivant : dans un tube flacon stérile, on introduit le milieu MEM 1,76 X en présence d'additifs (Glutamine 1%, Acides aminés non essentiels 1%, Pyruvate de sodium 1%, Fungizone 1% et Pénicilline/Streptomycine 1%), le sérum de veau foetal, la soude NaOH 0,1 N. On ajoute alors les fibroblastes isolés à partir d'explants de peau humaine à la concentration de $1,4 \times 10^5$ cellules pour 1 ml de milieu de culture.

On ajoute alors lentement, contre la paroi du tube de façon à observer l'apparition d'un nuage blanchâtre, un mélange volume/volume de collagène dans de l'acide acétique au 1/1000.

L'ensemble est alors mélangé avec précaution et réparti dans les puits d'une plaque de culture de 12 puits (type Costar référence 3512) à raison de 0.5ml de mélange par $cm^2$. La plaque de culture est ensuite placée dans un incubateur à 37°C avec 5% de $CO_2$.

Une fois formés après polymérisation du collagène, les dermes équivalents sont laissés adhérents au support de culture pendant 3 jours puis détachés du support pour que la contraction puisse démarrer. Ces dermes équivalents attachés sont sortis de l'incubateur pour effectuer les prises d'image en vue de la mesure de leur surface et ce pour chaque point de la cinétique de contraction (0, 4, 8 et 24 heures). Ils sont immédiatement remis dans l'incubateur entre chaque point de mesure.

L'évaluation de la contraction spontanée des dermes équivalents traités et témoin est réalisée en mesurant leur surface, à différents temps après le début de la contraction spontanée.

Pour cela, une image numérique est acquise pour chaque derme équivalent traité ou non traité au moyen d'une caméra (Caméra CCD -Iris Sony DXC -107P) et la surface est ensuite calculée sur chaque image au moyen d'un système d'analyse d'image (Zeiss Axiovision 3.0). A cette mesure de surface correspond un pourcentage de contraction égal au rapport des surfaces selon la formule :

$$\% \text{ contraction} = (Sp-Si)/Sp \times 100$$

où :

Sp représente la surface d'un puits de la plaque de culture ; elle correspond à la surface totale du derme équivalent avant contraction
Si représente la surface du derme équivalent à l'instant i de la cinétique de contraction.

c) Résultats

Comme illustré à la Figure ci-jointe, le taux de contraction du derme équivalent témoin est de 32% quatre heures après l'avoir détaché de son support. Il progresse à 42% après huit heures pour atteindre 54% après vingt-quatre heures.

L'association d'adénosine et sels de magnésium et potassium réduit ce pourcentage de contraction de 10% après quatre heures, 14% après huit heures et 19% après vingt-quatre heures, par rapport au témoin.

Ces valeurs sont bien supérieures à celles obtenues pour chacun des composés individuels testés.

Ce test démontre ainsi que l'association d'adénosine et de sels de magnésium et potassium entraîne une moindre contraction du derme équivalent, par rapport au témoin et au derme équivalent traité par ces composés séparément. Cet effet relaxant de l'association selon l'invention peut être mis à profit pour la préparation de compositions cosmétiques à effet anti-rides, en particulier pour lisser les rides d'expression.

**Exemple 2 : Composition cosmétique**

Cette composition est préparée de manière classique pour l'homme du métier. Les quantités données dans cet exemple sont indiquées en pourcentages pondéraux.

| | |
|---|---|
| Adénosine | 0,10% |
| Sulfate de magnésium | 0,05 % |
| Glycyrrhizinate dipotassique | 0,05 % |
| Acide stéarique | 3,00 % |
| Mélange de mono-stéarate de glycéryle et stéarate de polyéthylène glycol (100 OE) | 2,50 % |
| Stéarate de polyéthylène glycol (20 OE) | 1,00 % |
| Cyclopentadiméthylsiloxane | 10,00 % |
| Charges | 3,00 % |
| Huiles végétales | 7,00 % |
| Huiles synthétiques | 6,00 % |
| Conservateurs | 1,20 % |
| Diméthylsiloxane oxyéthyléné (16 OE) à extrémités méthoxy | 1,00 % |
| Gomme de silicone | 0,20 % |
| Copolymère acrylique en émulsion inverse (Simulgel 600 de SEPPIC) | 1,70 % |
| Alcool stéarylique | 1,00% |
| Eau        qsp | 100 % |

Cette crème est destinée à être appliquée sur le visage et le front pour atténuer les rides d'expression et décontracter le visage.

**Exemple 3 : Evaluation *in vivo* de l'effet sur les rides d'expression**

La composition de l'Exemple 2 été testée sur un panel de 40 femmes. Les résultats de l'auto-évaluation réalisée sont rassemblés dans le Tableau suivant.

| Paramètre évalué | % de réponses positives | |
|---|---|---|
| | t = 21 jours | t = 2 mois |
| Peau plus lisse | 95% | 97,5% |
| Traits moins crispés | 85% | 92,5% |
| Rides du front moins marquées | 62,5% | 60% |
| Sillon nasogénien moins marqué | 55% | 67,5% |
| Rides de la patte d'oie moins marquées | 72,5% | 75% |
| Rides inter-sourcillières moins marquées | 57,5% | 57,5% |

Il ressort de ce tableau que la composition selon l'invention a un effet positif sur les différentes rides d'expression et en particulier sur les rides de la patte d'oie.

**Revendications**

1. Composition comprenant, dans un milieu physiologiquement acceptable adapté à une application topique sur la peau, l'adénosine, au moins un sel de magnésium et au moins un sel de potassium.

2. Composition selon la revendication 1, **caractérisée en ce que** ledit sel est un sel organique formé à partir d'un contre-anion choisi parmi : un anion citrate, oxalate, acétate, gluconate, lactate, tartrate, maléate, benzoate, propionate, salicylate, ascorbate, formate, succinate, folinate, aspartate, phthalate, oléate, palmitate, stéarate, lauryl sulfate, lanolate, myristate, béhénate, caséinate, cyclamate, pantothénate, polyaminopolycarboxylate, thioglycolate, laurate, ricinoléate, pidolate, sorbate ou glycyrrhizinate.

3. Composition selon la revendication 1, **caractérisée en ce que** ledit sel est un sel inorganique formé à partir d'un contre-anion choisi parmi : un anion nitrate, sulfate, halogénure, carbonate, bicarbonate, hydroxyde, peroxyde, nitrure, sulfure, bisulfate, persulfate, glycérophosphate, hypophosphate ou borate.

4. Composition selon la revendication 3, **caractérisée en ce que** ledit sel de magnésium est le sulfate de magnésium.

5. Composition selon la revendication 2, **caractérisée en ce que** ledit sel de potassium est le glycyrrhizinate dipotassique.

6. Composition selon l'une quelconque des revendications 1 à 5, **caractérisée en ce qu'**elle renferme de 0,01 à 1% d'adénosine, par rapport au poids total de la composition.

7. Composition selon l'une quelconque des revendications 1 à 6, **caractérisée en ce qu'**elle renferme de 0,01 à 0,1% de sel de magnésium, par rapport au poids total de la composition.

8. Composition selon l'une quelconque des revendications 1 à 7, **caractérisée en ce qu'**elle renferme de 0,01 à 0,1% de sel de potassium, par rapport au poids total de la composition.

9. Procédé cosmétique pour réduire les rides et/ou détendre les traits et/ou décontracter la peau, comprenant l'application topique sur la peau d'une composition selon l'une quelconque des revendications 1 à 8.

10. Procédé selon la revendication 9, **caractérisé en ce que** lesdites rides sont des rides d'expression.

11. Procédé selon la revendication 10, **caractérisé en ce que** lesdites rides d'expression sont choisies parmi : les rides de la patte d'oie, les sillons nasogéniens, les rides inter-sourcillières et les rides du front.

12. Procédé selon l'une quelconque des revendications 9 à 11, **caractérisé en ce que** la composition est appliquée sur les zones du visage ou du front marquées par des rides d'expression et/ou sur les personnes présentant des rides d'expression.


**Claims**

1. Composition comprising, in a physiologically acceptable medium suitable for topical application to the skin, adenosine, at least one magnesium salt and at least one potassium salt.

2. Composition according to Claim 1, **characterized in that** said salt is an organic salt formed from a counter anion chosen from: a citrate, oxalate, acetate, gluconate, lactate, tartrate, maleate, benzoate, propionate, salicylate, ascorbate, formate, succinate, folinate, aspartate, phthalate, oleate, palmitate, stearate, lauryl sulphate, lanolate, myristate, behenate, caseinate, cyclamate, pantothenate, polyaminopolycarboxylate, thioglycolate, laurate, ricinoleate, pidolate, sorbate or glycyrrhizinate anion.

3. Composition according to Claim 1, **characterized in that** said salt is an inorganic salt formed from a counter anion chosen from: a nitrate, sulphate, halide, carbonate, bicarbonate, hydroxide, peroxide, nitride, sulphide, bisulphate, persulphate, glycerophosphate, hypophosphate or borate anion.

4. Composition according to Claim 3, **characterized in that** said magnesium salt is magnesium sulphate.

**5.** Composition according to Claim 2, **characterized in that** said potassium salt is dipotassium glycyrrhizinate.

**6.** Composition according to any one of Claims 1 to 5, **characterized in that** it contains from 0.01% to 1% of adenosine, relative to the total weight of the composition.

**7.** Composition according to any one of Claims 1 to 6, **characterized in that** it contains from 0.01% to 0.1% of magnesium salt, relative to the total weight of the composition.

**8.** Composition according to any one of Claims 1 to 7, **characterized in that** it contains from 0.01% to 0.1% of potassium salt, relative to the total weight of the composition.

**9.** Cosmetic process for reducing wrinkles and/or relaxing the features and/or decontracting the skin, comprising the topical application to the skin of a composition according to any one of Claims 1 to 8.

**10.** Process according to Claim 9, **characterized in that** said wrinkles are expression wrinkles.

**11.** Process according to Claim 10, **characterized in that** said expression wrinkles are chosen from: crow's-feet wrinkles, nasal grooves, wrinkles between the eyebrows and wrinkles on the forehead.

**12.** Process according to any one of Claims 9 to 11, **characterized in that** the composition is applied to the areas of the face or of the forehead that are marked with expression wrinkles, and/or to individuals with expression wrinkles.


**Patentansprüche**

**1.** Zusammensetzung, die in einem physiologisch akzeptablen Medium, das für eine topische Anwendung auf die Haut geeignet ist, Adenosin, mindestens ein Magnesiumsalz und mindestens ein Kaliumsalz enthält.

**2.** Zusammensetzung nach Anspruch 1, **dadurch gekennzeichnet, dass** das Salz ein organisches Salz ist, das mit einem Gegenion gebildet ist, das ausgewählt ist unter: Citrat, Oxalat, Acetat, Gluconat, Lactat, Tartrat, Maleat, Benzoat, Propionat, Salicylat, Ascorbat, Format, Succinat, Folinat, Aspartat, Phthalat, Oleat, Palmitat, Stearat, Laurylsulfat, Lanolat, Myristat, Behenat, Caseinat, Cyclamat, Pantothenat, Polyaminopolycarboxylat, Thioglycolat, Laurat, Ricinoleat, Pidolat, Sorbat oder Glycyrrhizinat.

**3.** Zusammensetzung nach Anspruch 1, **dadurch gekennzeichnet, dass** das Salz ein anorganisches Salz ist, das mit einem Gegenion gebildet ist, das ausgewählt ist unter: Nitrat, Sulfat, Halogenid, Carbonat, Bicarbonat, Hydroxid, Peroxid, Nitrid, Sulfid, Bisulfat, Persulfat, Glycerophosphat, Hypophosphat oder Borat.

**4.** Zusammensetzung nach Anspruch 3, **dadurch gekennzeichnet, dass** das Magnesiumsalz das Magnesiumsulfat ist.

**5.** Zusammensetzung nach Anspruch 2, **dadurch gekennzeichnet, dass** das Kaliumsalz das Dikaliumglycyrrhizinat ist.

**6.** Zusammensetzung nach einem der Ansprüche 1 bis 5, **dadurch gekennzeichnet, dass** sie 0,01 bis 1 % Adenosin, bezogen auf das Gesamtgewicht der Zusammensetzung, enthält.

**7.** Zusammensetzung nach einem der Ansprüche 1 bis 6, **dadurch gekennzeichnet, dass** sie 0,01 bis 0,1 % Magnesiumsalz, bezogen auf das Gesamtgewicht der Zusammensetzung, enthält.

**8.** Zusammensetzung nach einem der Ansprüche 1 bis 7, **dadurch gekennzeichnet, dass** sie 0,01 bis 0,1 % Kaliumsalz, bezogen auf das Gesamtgewicht der Zusammensetzung, enthält.

**9.** Kosmetisches Verfahren zur Verminderung von Falten und/oder um die Gesichtszüge zu entspannen und/oder die Haut zu entspannen, das die topische Anwendung einer Zusammensetzung nach einem der Ansprüche 1 bis 8 auf die Haut umfasst.

**10.** Verfahren nach Anspruch 9, **dadurch gekennzeichnet, dass** es sich bei den Falten um Mimikfalten handelt.

**11.** Verfahren nach Anspruch 10, **dadurch gekennzeichnet, dass** die Mimikfalten ausgewählt sind unter: Krähenfüßen, Nasenfurchen, Falten zwischen den Augenbrauen und Stirnfalten.

**12.** Verfahren nach einem der Ansprüche 9 bis 11, **dadurch gekennzeichnet, dass** die Zusammensetzung auf die Bereiche des Gesichts oder der Stirn aufgebracht werden, die Mimikfalten aufweisen, und/oder bei Personen, die Mimikfalten haben.

Evaluation de trois composés (adénosine, sulfate de magnésium et glycyrrhizinate dipotassique) seuls ou en mélange sur la contraction de dermes équivalents

**FIGURE UNIQUE**